# EUROPEAN PATENT SPECIFICATION

(11) **EP 1 384 191 B1**
(45) Date of publication and mention of the grant of the patent: **30.08.2006**
(21) Application number: 01995192.0
(22) Date of filing: 16.11.2001
(51) Int. Cl.: G06F 19/00

(54) **A SYSTEM AND METHOD FOR ANNOTATING PATIENT MEDICAL INFORMATION**
SYSTEM UND VERFAHREN ZUM KOMMENTIEREN VON MEDIZINISCHEN PATIENTENINFORMATIONEN
SYSTEME ET PROCEDE D'ANNOTATION D'INFORMATIONS MEDICALES RELATIVES A UN PATIENT

(30) Priority: 17.11.2000 US 249575 P
(43) Date of publication of application: 28.01.2004
(73) Proprietor: Draeger Medical Systems, Inc., Danvers, MA 01923 (US)
(72) Inventor: AUER, John, E., Ipswich, MA 01938 (US); RUTLEDGE, Jolyn, Amesbury, MA 01913 (US)
(74) Representative: Wilding, Frances Ward
(86) International application number: PCT/US2001/043751
(87) International publication number: WO 2002/041230

(56) References cited:
- EP-A- 0 676 709
- WO-A-98/29790
- WO-A-98/59487
- US-A- 5 682 526

## Description

### Field of the Invention

This invention is related to the processing and displaying of medical information, and more particularly to processing and displaying of patient medical parameters along with user-entered text information in a network environment.

### Background of the Invention

In hospitals and other health care environments, it is often necessary or desirable to collect and display a variety of medical data associated with a patient. Such information may include vital sign data, care unit data, diagnosis and treatment procedures, ventilator information, and other parameter data associated with a given patient. Presently, such information is often provided via a chart attached to a patient's bedside or at an attendant's station. However, such physical charts are cumbersome to view, and often do not include the most up-to-date medical information associated with the patient, such as laboratory test results, trend analysis information or annotation data. This problem is exacerbated due to the fact that such medical data arrives from multiple sources and at various times. Furthermore, present charts are not adapted to enable a care giver to easily access, view, or determine the results of multiple medical tests or other data associated with the patient Still further, certain specific medical data or lab results may warrant particular annotated information that the physician or other care giver desires to be maintained within the overall patient information, and associated with a particular time period. Consequently, a need exists for a faster, more effective and user friendly means for accessing, correlating, displaying and annotating patient medical information derived from a plurality of sources.

USP 5,682,256 recites a method and system for organizing, recording and displaying medical patient care information. The system allows a user to define and organize the information that may be stored about a patient. The system allows a user to set a hierarchy of information parameters. The layout and format of flowsheets for the system may be customized.

The invention is defined in the independent claims, to which reference should now be made. Preferred features are set out in the subclaims.

### Brief Description of the Drawings

In the drawings:
Figure 1 is a block diagram of a communication network with various devices, according to the principles of the invention.
Figures 2 represents a flow diagram of a system for annotating selected medical parameter data according to the present invention.
Figures 3A-3E provide exemplary illustrations of user interface displays for annotating selected medical parameter data according to an aspect of the present invention.
Figure 4 is an exemplary illustration of a block diagram of a server having functionality in accordance with the present invention.

### Detailed Description

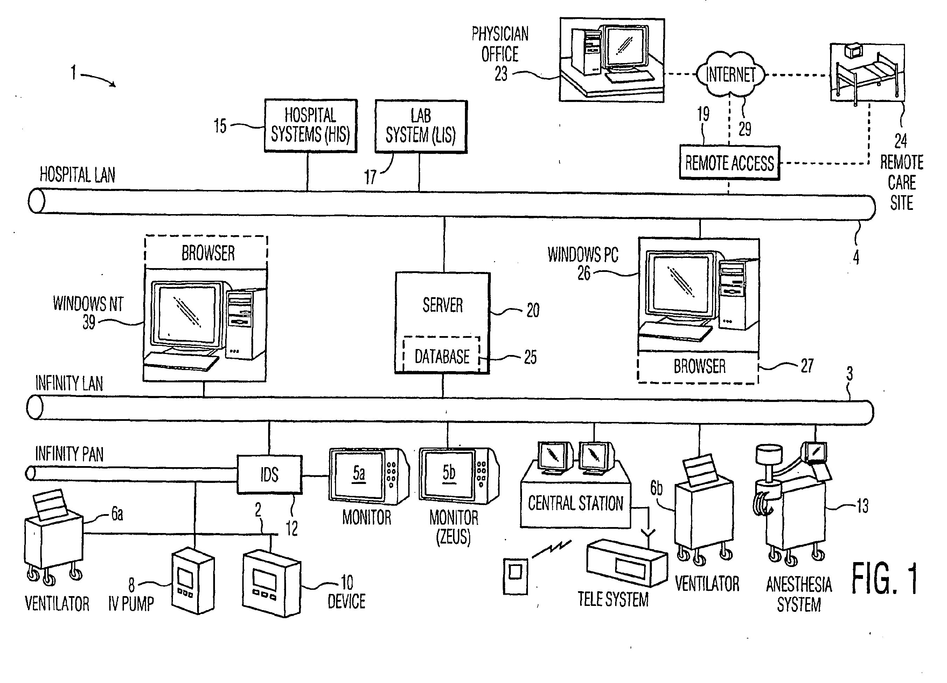

Figure 1 is an exemplary block diagram of a communication network according to the principles of the present invention. Throughout the document, like reference numerals are used to indicate like parts. As shown in Fig. 1, communication network 1 is represented by an IP (Internet Protocol) compatible network with a hierarchy of local area and wide area networks interconnected together. It is to be noted that although the present exemplary hospital or medical network is an IP compatible network, other types of networks such as, but not limited to optical or wireless networks, using other computing protocols such as, but not limited to, for example, X.25, frame relay, IBM SNA etc., may also be used, as one skilled in the art can readily appreciate. In addition, although the exemplary network described is a hierarchical network, this is not required by the present invention. Any type of network architecture that provides communication connectivity among the devices on the network may be used.

As shown on Fig. 1, the first level of the exemplary hierarchical network 1 comprises a Medical Interface Bus (MIB) 2. A MIB is a well-known medical industry standard for locally connecting medical devices together. As shown in Fig. 1, MIB 2 is typically used to interconnect medical devices in a patient's room to administer care to a particular patient and to monitor the particular patient. Various medical devices may be connected via MIB 2; examples shown in Fig. 1 comprise a ventilator 6a, IV (Intravenous) Pump 8 or other medical equipment 10.

MIB 2 is typically connected to a second level LAN network 3 through an Interface Docking Station (IDS) device 12, for interfacing to Ethernet-compatible LAN network 3. The higher-level LAN 3 may be for example, an Infinity LAN, marketed by Siemens Medical System. This higher-level LAN 3 is typically, though not necessarily, used by a particular department within a hospital, such as an intensive care department or surgery department, etc., depending on the size of the organizations.

Although not shown in Fig. 1, more than one MIB may be connected to the second level LAN 3, so that more than one patient may be monitored or given care through LAN 3. In addition, medical devices may be connected directly to higher-level LAN 3. For example, as shown in Fig. 1, a ventilator 6b and an anesthesia system 13 are connected directly to LAN 3, without the need to go through a MIB.

Furthermore, LAN 3 may be interconnected to a Hospital LAN backbone 4 which also is Ethernet compatible. This backbone network 4 provides communication connectivity between various departments within a hospital or medical organization; for example, connecting hospital administrative systems 15 together with laboratory systems 17. In addition, the Hospital LAN 4 has a remote access gateway 19 which provides remote, secured access from, for example, a remote doctor's office 23 or a remote care site 24, to the various systems and devices on network 1, through for example, Internet 29. Alternatively, a remote site may also access the remote access gateway 19 directly through, for example, a dial-up telephone port, ADSL, or other types of private connection. Remote access gateway 19 may also be part of server 20, to be described below, instead of standing alone, as well know in the art.

According to the principles of the present invention, a central server 20 resides on LAN 3 for gathering and processing data from the peripheral medical devices or facilities coupled to LAN 3 or hospital LAN 4, including medical parameters such as lab results supplied via lab system 17 connected through an HL7 interface, for example. Additional medical parameter data including cardiology, hemodynamic, ventilation and neurology category data may also be acquired from any number of medical devices such as those shown in Figure 1 and may be obtained at server 20 using various interface protocols including HL7 or ASTM messaging, for example. The acquired medical parameters associated with a given patient, including vital signs and laboratory test results, are acquired from the medical devices on network 1 for display and control. One skilled in the art can readily recognize that server 20 may reside at any level of the hierarchy of network 1, since all the different levels of LANs (e.g., 3, or 4), as well as remote sites in Fig. 1 are interconnected together. An example of server 20, is a ChartAssist™ server, marketed by Siemens Medical System. The server may be hosted, for example, by a computer system that is capable of running Microsoft NT operating system.

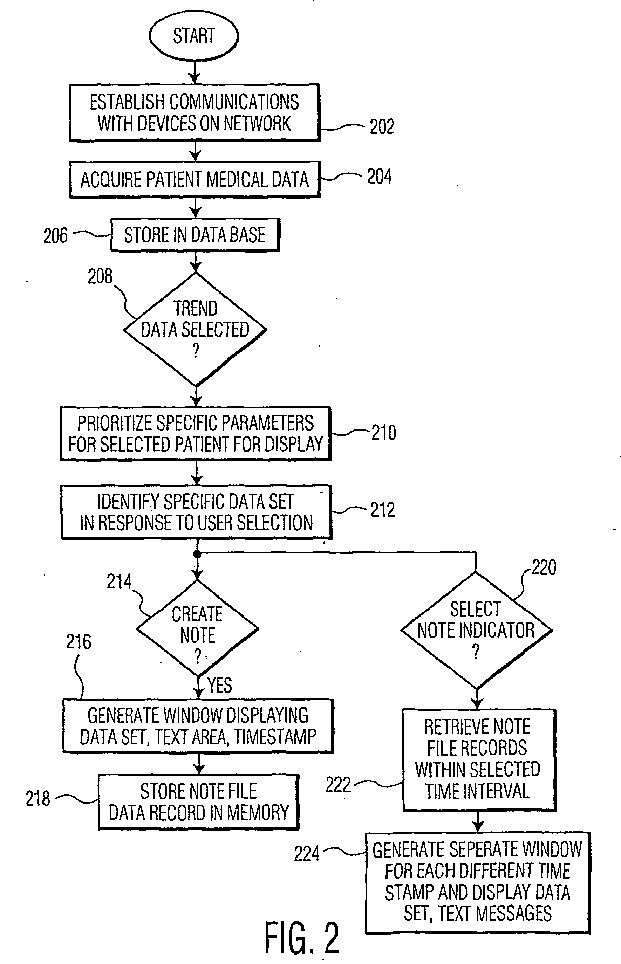

Fig. 2 shows in flow chart form, functions that may be performed by server 20 in conjunction with the user interface software resident on a web browser 27 of a client computer 26 configured to retrieve and display medical information associated with a selected patient along with annotated information corresponding to specific patient parameter data according to the present invention. Server 20 first establishes communications with devices on the network as shown in step 202. This is done, for example, by using IP protocol and the known IP device address for each device on the network 1, in conjunction with any higher application-layer protocols, as well known in the art.

Once communications are established between server 20 and the other devices, server 20 starts to acquire parameters that are being monitored and settings selected for various devices. A communication processing module or software program operates to acquire the patient data including the monitored parameters and collate the information for storage in a data base. As previously mentioned, such parameter data may be obtained through an HL7 interface with LIS 17, or via ASTM or MIB point of care (POC) medical devices depicted in Figure 1.

Medical parameter data including cardiology, lab results, hemodynamic, ventilation and neurology category data may be continuously or periodically acquired and correlated with a given patient for storage in relational data base 25 within server 20. Data base 25 may be of the type used for storing relational data such as the Microsoft SQL server. The acquired data may include time stamp information or other information indicative of the date and time associated with the acquired data.

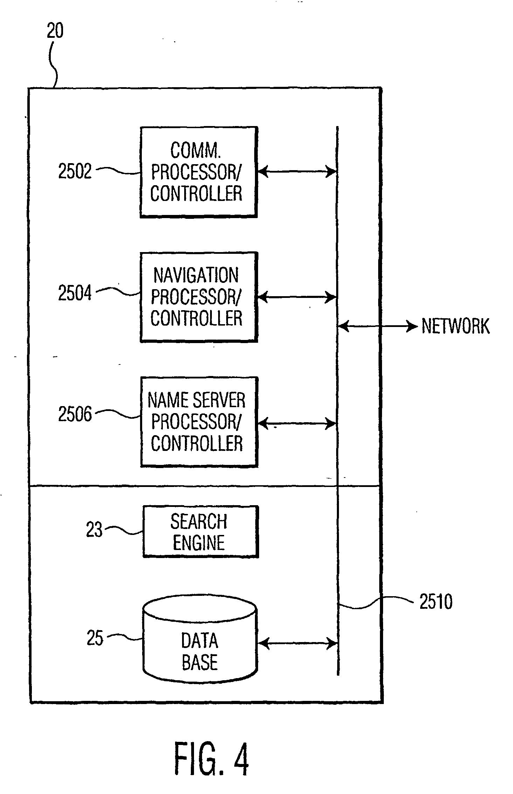

Server 20 is therefore capable of collating and formatting medical data to be compatible with, for example, HTML (HyperText Mark-up Language) programming language for displaying data on a web browser having a graphical user interface (GUI) component. The server is also responsive to, for example, HTTP (HyperText Transfer Protocol) commands originated from a user's web browser for making a request. Figure 4 shows a block diagram of an exemplary embodiment of the server 20 which operates to manage, collate, search and update the data base 25 containing patient medical information. Program elements or processors operative to carry out instructions for performing the various functions described herein include communications processing module 2502 that acquires the patient data including the monitored data parameters associated with corresponding patients and collates the information for storage in data base 25. Processor/controller 2504 operates in conjunction with the web browser and display generator software to prioritize the acquired patient medical data parameters for displaying certain patient medical data associated with a particular patient in a desired order and format in response to user selection of a particular application. Name server processor 2506 associates unique identifiers (Ids) with each node connected to the system network and with each patient in the system in order to track and update patient information throughout the system. Input/output data and control signals are used to communicate between the various processors as well as to interface with the data base 25 and search engine 23 and with the network via communication line 2510.

In one aspect of the present invention, a user may use a Microsoft Windows compatible PC 26 or Windows NT compatible PC 39 as shown in Fig. 1, or any other computers capable of running a menu generating program such as a web browser program (e.g., Microsoft Internet Explorer or Netscape Navigator, etc.) to view the aforementioned category type medical data associated with a given patient. That is, a user may use a web browser on any computer, as long as a communication connection can be made to server 20, to make request and view information acquired and stored in data base 25. This is advantageous, since a doctor may for example, gain access to medical parameter data from, for example, a remote physician's office 23, without having to access a dedicated terminal. Of course, a user can simply use a keyboard and/or a mouse or any other user interface devices to enter a user selection or request on a user computer, as is known in the art. The user interface contains functionality for displaying patient medical information associated with a selected patient while navigating between different applications operative to retrieve-and display different medical data associated with the selected patient. The user interface further includes functionality for selecting a subset (i.e. data set) of data from the displayed data associated with a given time period and for displaying that data set along with user entered textual information. Such functionality includes a browser containing a display generator module for displaying a composite window containing both the specific data set parameter data selected and annotated text information associated with a selected patient.

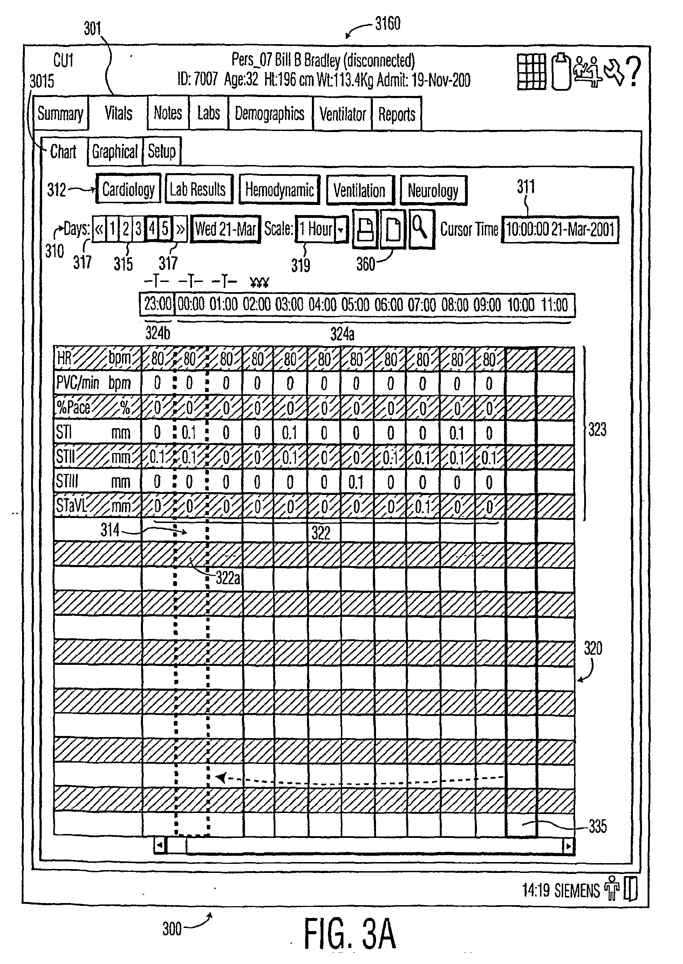

Fig. 3A shows an example of how medical parameter data associated with particular monitored parameters may be retrieved and displayed on a web browser of a user computer 26 along a timeline spanning multiple days to enable a user of the system to view and annotate trend data, according to the present invention. As shown, a display window 300 comprises a navigator panel portion 310 and a results display window portion 320. Display window 320 contains particular medical parameter data labeled generally as 322 displayed in a predetermined format along a timeline 324 in response to a user request for access to particular medical parameter data associated with a given patient.

In the exemplary embodiment shown in Figure 3A, the medical parameter data 322 and associated data labels 323 are displayed in display window 320 in tabular or chart format in response to user selection of Vitals tab 301-> Chart subtab 3015. Selection of one of the icons labeled generally as 312 and corresponding to particular medical parameters associated with a corresponding one of cardiology, lab results, hemodynamic, ventilation and neurology categories, causes the user interface to request a search of the data base to obtain those particular medical parameters within the category selected that are associated with the selected patient. Due to the large amount of patient data that accumulates during a patient's stay in the hospital, an undesirably large amount of medical parameter data meeting the search criteria may be displayed to the user. Advantageously, the user interface apparatus according to the present invention further restricts the medical parameter data displayed to a subset of that data corresponding to a user selected date range, which is then displayed along timeline 324.

Display navigator panel 310 comprises a scrollable, user selectable day indicator panel 315 containing the entire number of days (i.e. calendar days) that a patient has been admitted according to the data base information associated with that given patient. In an exemplary embodiment, five days (1, 2, 3, 4, 5) are displayed via day indicator panel 315 with directional control selectors 317 embodied in the form of left and right arrow buttons on either side of the display indicator panel to enable a user to scroll through the entire range of days. User selection of a particular day within the day indicator panel day range causes the search engine to retrieve from the data base all medical parameter data for a given patient associated with the selected day, the immediately preceding day, and immediately succeeding day, that also meet all other search criteria (e.g. category of medical parameter data).

As shown in Figure 3A, medical parameter data 322 is displayed to the user in tabular form across day boundaries in response to user selection of a particular day (e.g. Day 5) within day indicator panel 315. The user interface operates to generate a timeline display 324 having a first portion 324a associated with the current or selected day and a second portion 324b associated with the previous or next day. In a particular embodiment, the timeline 324 is segmented into predetermined intervals T of equal duration. These intervals are scalable in user selectable increments of 15 minutes, 1 hour, 2 hours, 4 hours or 8 hours based on user selection of scale panel 319 and formatted for display in window 320. The timeline display includes indicia in hour/minute (hh:mm) format enabling a user to identify the particular time associated with particular corresponding displayed parameter data, as well as enabling a user to view or determine trends associated with the patient medical data. The retrieved medical data is prioritized, collated and displayed in a desired order in accordance with the search criteria. In the embodiment shown in Figure 3A, medical parameter data associated with particular medical parameters 323 comprising Heart Rate (HR) PVC/min, %Pace, STI, STII, STIII and STaVL are displayed in descending order along a first column while the corresponding data associated with each of the parameters are displayed in time sequence fashion along the horizontal or row. The data is aligned with the timeline display to associate a temporal period with a given column's parameter data. The right most data displayed via the web browser represents the most recent medical parameter data. Each column defines a given subset 322a of data selectable by the user by positioning a cursor over that desired column and selecting the column (via a mouse click, for example). A separate cursor time display window 311 responsive to user selection of a given column (for example, column 335) displays the date and time associated with the selected position of the cursor.

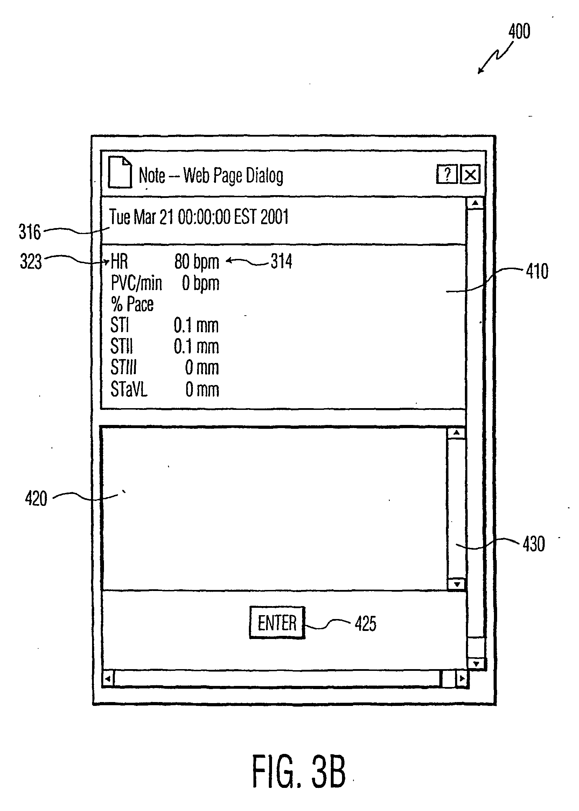

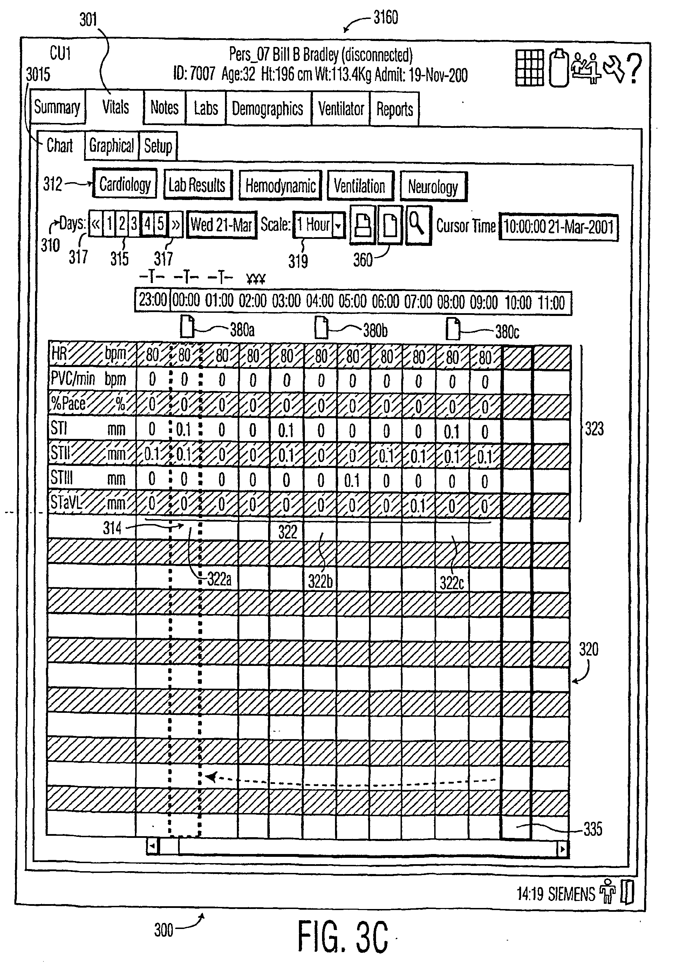

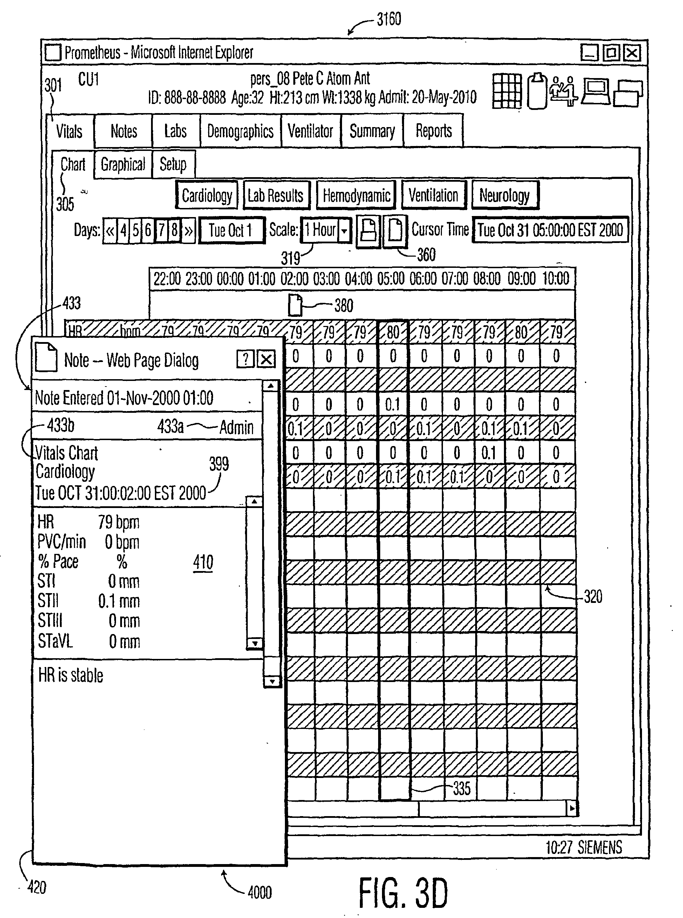

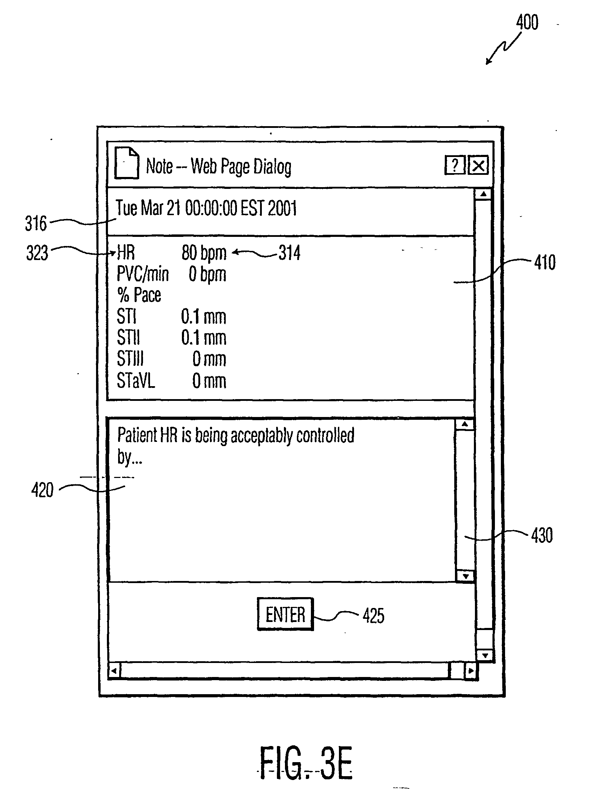

Referring now to Figures 3A-3D in conjunction with Figure 2, there is shown a system and method for enabling a user to annotate selected trend data with text while automatically capturing specific medical data including vital sign parameters within a selected data set in addition to time stamp information within a note file. As previously discussed, user selection of the Vitals -> Chart tabs for a given patient 3160 within the network results in medical parameter data 322 displayed to the user in tabular form as shown in Figure 3A. A given column or data set 322a defining a set of specific displayed parameter is then selected via a cursor, and create note icon 360 is then selected to generate window 400 associated with a particular time period as shown in Figure 3B. Window 400 comprises parameter display portion 410 and text entry/display portion 420 which is initially blank. Each of the particular data points 314 associated with selected data set 322a along with their respective parameter labels 323 including their units of measure are automatically copied into display portion 410 and formatted for display to the user in the same order as shown in display 300 (Fig. 3A). The specifically identified displayed parameters have values representing a trend point in the patient medical data derived from a patient monitoring device. Text entry/display portion 420 provides an area enabling a user to enter textual information concerning the identified specific displayed parameters. This enables a user of the system, such as a physician, nurse or other caregiver having appropriate access, to enter additional text that corresponds to the particular medical parameter data selected for a particular time period. Such electronic annotation of trend information facilitates easy retrieval and review of particular data, while eliminating the need for transcribing information onto multiple pages, writing in margins or on corners of documents (e.g. lab charts). Such information may be entered via a computer keyboard, for example. Figure 3E shows user entry of text information within entry/display portion 420. User selection of control button 425 causes the system to create and store in a data store memory such as a data base record data representing the user-entered text message and the identified specific displayed parameter data 314 and labels 323. Time stamp information is also included within window 410 (Figures 3B and 3E) and in the stored data base record (Figure 3D) to provide detailed cursor time and date information corresponding to the selected parameter data set. In addition, date time-stamp information 433 (Figure 3D) representing the note file create date and time and also including user create information 433a and category information 433b is stored in the data base record. In a particular embodiment, the information stored in the data base is processed as an HTML string and formatted so as to enable proper display via note indicator icon 380 (Figure 3D). In a further exemplary embodiment, the data base record is indexed by the date time stamp information 316.

Display generator software operates in response to entry of the textual information and selection of control button 425 for displaying an indicator 380 positioned above the specific data set 322A selected for providing a visual indication that a note file had been created for that particular data set. Figure 3C provides an exemplary illustration of screen display 300 containing note file indicators 380a, 380b, and 380c indicative of the creation of note files associated, respectively, with a corresponding one of data sets 322a, 322b, and 322c. Subsequent note files can be created and annotated for each of the data set columns displayed in window 320 in the manner described herein.

Figure 3D provides an exemplary illustration of a note file window 4000 corresponding to the vital sign parameter data set identified by note indicator 380. As shown in Figure 3D, user selection of note indicator 380 causes the search engine to retrieve all note file data base records for notes within the selected time interval as provided within the user selectable scale increment 319. That is, the search engine will retrieve and display all note file records in separate note windows for all note files that were created within the predetermined time interval (e.g. 1 hour) of the specific data parameter set selected. Note further that software processes operate to concatenate data information parameter display portion 410 and text entry display portion 420 into a single note window in the event that two note files are created for the same specific display parameter set. In addition, note indicator 380 may be implemented as an active display element or hot area such that cursor movement onto the hot area about indicator area 380 results in a display of summary data associated with the user annotated text. This may be, for example, the first 10 characters of the text entered within the note file and may be displayed to the user in response to cursor movement or selection of indicator 380. In this manner, a user may quickly derive annotation information from the display without selecting and viewing the entire annotated text file that is stored in memory.

As a further example, Figure 2 provides a flow chart illustration for performing the functions associated with annotating selected trend data points with entered text while automatically capturing specific medical data parameters within a note file. As previously mentioned, upon establishing communications with devices on the network (step 202) server 20 begins acquiring parameters that are being monitored in settings selected for various devices connected to the network for storage in the data base. Trend data may then be selected by navigation of the various applications within the system such as by selecting the vitals -> chart tab functions for prioritizing specific parameters for a selected patient for display to the user (step 210). User selection of a particular data set by, for example, a mouse click, operates to allocate an identifier attribute to the specific set of displayed parameters in response to this selection. As shown in Figure 3A, this attribute is illustrated as a boxed area, however other attributes are also contemplated including different color indicators, geometric shapes, symbols and the like.

Selection of create note icon 360 (step 214) causes server software to create note window 400 displaying the user selected specific display parameters and including the text area for enabling user entry of textual information (step 216). Upon user annotation within display entry portion 420 and processing of note window 400, a data base record containing the user selected parameters, date time stamp, annotated text information, and note create time stamp information is stored as an HTML string in the data base. Note file indicator 380 is then displayed to the user for that specific display parameter set. Selection of note indicator 380 (step 220) causes the system to retrieve all file records for notes within the selected time interval 319 (see Figure 3D) and to generate a separate note file window 4000 displaying the user selected parameters and textual information for each data base record within the predetermined interval 319 having a different date time stamp 399 associated with specific display parameters.

It is to be understood that the embodiments and variations shown and described herein are for illustrations only and that various modifications may be implemented by those skilled in the art without departing from the scope of the invention.

## Claims

1. A user interface apparatus for use in a network compatible system (1) for displaying medical information derived from a plurality of sources, said user interface apparatus comprising:
a communication processor (20) for acquiring patient medical data (204, 2502);
a processor (20) for prioritizing acquired patient medical data for display (210) in a desired order and identifying specific displayed parameters of said data in response to a user selection command (212, 2504) ; and
a display generator for generating a window (320) in response to user activation of a displayed icon (312), said window automatically including said identified specific displayed parameters (322) and also including user entered messages.

2. The apparatus of claim 1, further comprising a memory (20) for storing a file of data representing said user-entered text messages (218) and said identified specific displayed parameters (314).

3. The apparatus of claim 1, wherein said apparatus permits a user to enter text message annotations (420) conceming said identified specific displayed parameters (314).

4. The apparatus of claim 1, wherein said identified specific displayed parameters (314) are values representing a trend point in patient medical data derived from a patient monitoring device.

5. The apparatus of claim 1, wherein said window automatically includes said identified specific displayed parameters (314) together with their associated parameter labels and units of measure (323) and
said apparatus automatically captures at least one of, (a) a vital sign parameter and (b) time stamp data (433), for display in said window.

6. The apparatus of claim 2, wherein
said file of data representing said user-entered text messages and said identified specific displayed parameters comprises an HTML string and
said window includes time stamp information associated with said identified specific displayed parameters (314) and time stamp information associated (433) with creation of the note file.

7. The apparatus of claim 1, wherein said displayed icon (312) includes an active area responsive to a cursor incident thereon for displaying a subset of the text messages contained in said window (420).

8. A network compatible method for annotating medical information displayed to a user comprising:
acquiring patient medical data (204) for storage in a data base (206);
prioritizing acquired patient medical data for display in a desired order (210);
identifying specific displayed parameters of said data in response to a user selection command (212); and
generating a window in response to user activation of a displayed icon, said window automatically including said identified specific displayed parameters and also including user entered text messages (216).

9. The method of claim 8, further comprising
storing in memory a note file representing said identified specific displayed parameters (314) and said user entered text messages (218) and
providing a visual indicator (380) associated with said specific displayed parameters (314) indicative of said stored note file.

10. The method of claim 8, wherein
the step of generating said window in response to user activation of a displayed icon further comprises determining whether a note file already exists for said specific displayed parameters (314) and
the specific displayed parameters (314) are values representing a trend point in patient medical data derived from a patient monitoring device.

11. The method of claim 8, wherein
said method is for displaying and annotating medical parameter data derived from a plurality of sources;
said activity of acquiring acquires medical parameters (204) associated with a patient on a periodic basis;
said activity of prioritizing is in response to a first user command;
said activity of identifying identifies a specific set of data parameters within said displayed medical parameters (212) in response to a second user command; and
further comprising the activity of displaying in a first window said specific set of data parameters (410) and in a second window a text field for annotating textual information (420) corresponding to said specific set of data parameters in response to a third user command.

12. The method of claim 11, further comprising
storing in memory a note file representing said first and second window displays (218) and providing an indicator associated with said specific displayed parameters (314) indicative of said stored note file.

13. The method of claim 11, wherein
the step of identifying a specific set of data parameters (212) within said displayed medical parameters comprises identifying at least one of (a) a vital sign parameter and (b) time stamp data, for display.

14. The apparatus of claim 1, wherein said window further includes an area for entering user defined text messages.

15. The system of claim 14, further comprising a user-selectable icon displayable in response to user creation of a note file for indicating that a note file has been created, said note file representing said user-entered text message (218) and said identified specific displayed parameters (314).

## Patentansprüche

1. Benutzerschnittstelleneinrichtung zum Gebrauch in einem System (1), das mit einem Netz kompatibel ist und medizinische Information darstellt, die aus zahlreichen Quellen stammt, wobei die Benutzerschnittstelleneinrichtung umfasst:
einen Kommunikationsprozessor (20), der medizinische Patientendaten (204, 2502) erfasst;
einen Prozessor (20), der erfasste medizinische Patientendaten für die Darstellung (210) in einer gewünschten Reihenfolge priorisiert und bestimmte dargestellte Parameter der Daten abhängig von einem Benutzerauswahlbefehl (212, 2504) kennzeichnet; und
einen Darstellungsgenerator, der ein Fenster (320) anhängig von der Aktivierung eines dargestellten Icons (312) durch einen Benutzer erzeugt, wobei das Fenster automatisch die **gekennzeichneten** bestimmten Darstellungsparameter (322) enthält sowie vom Benutzer eingegebene Meldungen.

2. Einrichtung nach Anspruch 1, zudem umfassend einen Speicher (20), der eine Datei aus Daten speichert, die die vom Benutzer eingegebenen Textmeldungen (218) darstellen sowie die **gekennzeichneten** bestimmten Darstellungsparameter (314).

3. Einrichtung nach Anspruch 1, wobei es die Einrichtung einem Benutzer erlaubt, Textmeldungskommentare (420) einzugeben, die die **gekennzeichneten** bestimmten Darstellungsparameter (314) betreffen.

4. Einrichtung nach Anspruch 1, wobei die **gekennzeichneten** bestimmten Darstellungsparameter (314) Werte sind, die einen Trendpunkt in den medizinischen Daten des Patienten darstellen, die von einer Patientenüberwachungsvorrichtung abgeleitet sind.

5. Einrichtung nach Anspruch 1, wobei das Fenster automatisch die **gekennzeichneten** bestimmten Darstellungsparameter (314) zusammen mit ihren zugehörigen Parameterbezeichnungen und Maßeinheiten (323) enthält, und
die Einrichtung automatisch mindestens entweder einen a) Lebenszeichenparameter oder b) Zeitangabedaten (433) für die Darstellung im Fenster erfasst.

6. Einrichtung nach Anspruch 2, wobei
die Datei mit Daten, die vom Benutzer eingegebene Meldungen darstellen, und die **gekennzeichneten** bestimmten Darstellungsparameter eine HTML-Zeichenkette enthalten, und
das Fenster Zeitangabeinformation enthält, die zu den **gekennzeichneten** bestimmten Darstellungsparametern (314) gehört, und Zeitangabeinformation, die der Erzeugung der Meldungsdatei zugeordnet (433) ist.

7. Einrichtung nach Anspruch 1, wobei das dargestellte Icon (312) eine aktive Fläche enthält, die auf einen dorthin geführten Cursor anspricht und eine Untermenge der Textmeldungen darstellt, die im Fenster (420) enthalten sind.

8. Netzkompatibles Verfahren zum Kommentieren medizinischer Information, die einem Benutzer angezeigt wird, umfassend:
das Erfassen von medizinischen Patientendaten (204), die in einer Datenbank (206) gespeichert werden;
das Priorisieren der erfassten medizinischen Patientendaten für die Darstellung in einer gewünschten Reihenfolge (210);
das Kennzeichnen bestimmter Darstellungsparameter der Daten aufgrund eines Benutzerauswahlbefehls (212); und
das Erzeugen eines Fensters aufgrund einer Aktivierung eines dargestellten Icons durch den Benutzer, wobei das Fenster automatisch die **gekennzeichneten** bestimmten Darstellungsparameter enthält und zudem vom Benutzer eingegebene Textmeldungen (216).

9. Verfahren nach Anspruch 8, zudem umfassend:
das Ablegen einer Meldungsdatei im Speicher, die die **gekennzeichneten** bestimmten Darstellungsparameter (314) und die vom Benutzer eingegebenen Textmeldungen (218) repräsentiert, und
das Bereitstellen einer sichtbaren Anzeige (380), die den bestimmten Darstellungsparametern (314) zugeordnet ist und die gespeicherte Meldungsdatei bezeichnet.

10. Verfahren nach Anspruch 8, wobei
der Schritt des Erzeugens des Fensters abhängig davon, dass der Benutzer ein dargestelltes Icon aktiviert, zudem die Feststellung umfasst, ob für die bestimmten Darstellungsparameter (314) bereits eine Meldungsdatei vorhanden ist; und
die bestimmten Darstellungsparameter (314) Werte sind, die einen Trendpunkt in den medizinischen Daten des Patienten darstellen, die von einer Patientenüberwachungsvorrichtung abgeleitet sind.

11. Verfahren nach Anspruch 8, wobei
das Verfahren dem Darstellen und Kommentieren medizinischer Parameter dient, die aus zahlreichen Quellen abgeleitet werden;
der Vorgang des Erfassens zu einem Patienten gehörende medizinische Parameter (204) periodisch erfasst;
der Vorgang des Priorisierens aufgrund eines ersten Benutzerbefehls erfolgt;
der Vorgang des Kennzeichnens eine bestimmte Menge Datenparameter innerhalb der dargestellten medizinischen Parameter (212) aufgrund eines zweiten Benutzerbefehls kennzeichnet; und
zudem in einem ersten Fenster der Vorgang des Darstellens der bestimmten Menge Datenparameter (410) und in einem zweiten Fenster das Darstellen eines Textfelds für kommentierende Textinformation (420), die zu der bestimmten Menge Datenparameter gehört, aufgrund eines dritten Benutzerbefehls erfolgt.

12. Verfahren nach Anspruch 11, zudem umfassend:
das Ablegen einer Meldungsdatei im Speicher, die die ersten und zweiten Fensterdarstellungen (218) repräsentiert, und das Bereitstellen einer Anzeige, die den bestimmten Darstellungsparametern (314) zugeordnet ist und die gespeicherte Meldungsdatei bezeichnet.

13. Verfahren nach Anspruch 11, worin
der Schritt des Kennzeichnens einer bestimmten Menge von Datenparametern (212) innerhalb der dargestellten medizinischen Parameter das Kennzeichnen mindestens entweder eines a) Lebenszeichenparameters oder b) von Zeitangabedaten für die Darstellung umfasst.

14. Einrichtung nach Anspruch 1, wobei das Fenster zudem einen Bereich enthält, der der Eingabe benutzerdefinierter Textmeldungen dient.

15. System nach Anspruch 14, zudem umfassend ein vom Benutzer wählbares Icon, das dargestellt werden kann, wenn der Benutzer eine Meldungsdatei erzeugt hat, und anzeigt, dass eine Meldungsdatei erzeugt ist, wobei die Meldungsdatei die vom Benutzer eingegebene Textmeldung (218) und die **gekennzeichneten** bestimmten Darstellungsparameter (314) repräsentiert.

## Revendications

1. Appareil formant interface utilisateur pour utilisation dans un système (1) compatible avec le réseau pour afficher des informations médicales obtenues d'une pluralité de sources, ledit appareil formant interface utilisateur comprenant :
un processeur de communication (20) pour acquérir des données médicales (204, 2502) se rapportant au patient;
un processeur (20) pour une disposition prioritaire de données médicales acquises se rapportant au patient pour l'affichage (210) dans l'ordre souhaité et pour identifier les paramètres spécifiques affichés desdites données en réponse à une commande de sélection utilisateur (212, 2504) ; et
un générateur d'affichage pour produire une fenêtre (320) en réponse à l'activation par l'utilisateur d'une icône affichée (312), ladite fenêtre comprenant automatiquement lesdits paramètres spécifiques affichés identifiés (322) et comprenant également des messages entrés par l'utilisateur.

2. Appareil selon la revendication 1, comprenant en outre une mémoire (20) pour stocker un dossier de données représentant lesdits messages de texte (218) entrés par l'utilisateur et lesdits paramètres spécifiques affichés identifiés (314).

3. Appareil selon la revendication 1, où ledit appareil permet à un utilisateur d'entrer des annotations de messages de texte (420) concernant lesdits paramètres spécifiques affichés identifiés (314).

4. Appareil selon la revendication 1, où lesdits paramètres spécifiques affichés identifiés (314) sont des valeurs représentant un point de tendance dans les données médicales du patient obtenues d'un dispositif de surveillance du patient.

5. Appareil selon la revendication 1, où ladite fenêtre comprend automatiquement lesdits paramètres spécifiques affichés identifiés (314) ensemble avec leurs étiquettes de paramètres associées et unités de mesure (323) et
ledit appareil saisit automatiquement au moins l'un parmi (a) un paramètre de signe vital et (b) des données d'estampille temporelle (433), pour l'affichage dans ladite fenêtre.

6. Appareil selon la revendication 2, où ledit dossier de données représentant lesdits messages de texte entrés par l'utilisateur et lesdits paramètres spécifiques affichés identifiés comprennent une chaîne HTML et
ladite fenêtre comprend des informations d'estampille temporelle associées auxdits paramètres spécifiques affichés identifiés (314) et des informations d'estampille temporelle associées (433) à la création du dossier de notes.

7. Appareil selon la revendication 1, où ladite icône affichée (32) comprend une zone active réagissant à un curseur incident sur celle-ci pour afficher un sous-ensemble de messages texte contenus dans ladite fenêtre (420).

8. Procédé compatible avec le réseau pour annoter des informations médicales affichées pour un utilisateur, comprenant les étapes consistant à :
acquérir des données médicales (204) se rapportant au patient pour le stockage dans une base de données (206) ; disposées selon la priorité des données médicales acquises se rapportant au patient pour l'affichage dans un ordre souhaité (210) ;
identifier des paramètres spécifiques affichés desdites données en réponse à une commande de sélection (212) par l'utilisateur ; et
produire une fenêtre en réponse à l'activation par l'utilisateur d'une icône affichée, ladite fenêtre comprenant automatiquement lesdits paramètres spécifiques affichés identifiés et comprenant également des messages texte (216) entrés par l'utilisateur.

9. Procédé selon la revendication 8, comprenant en outre les étapes consistant à
stocker dans la mémoire un dossier de notes représentant lesdits paramètres spécifiques affichés identifiés (314) et lesdits messages texte (218) entrés par l'utilisateur et réaliser un indicateur visuel (380) associé auxdits paramètres spécifiques affichés (314) indicateurs dudit dossier de notes stocké.

10. Procédé selon la revendication 8, dans lequel l'étape consistant à produire ladite fenêtre en réponse à l'activation par l'utilisateur d'une icône affichée comprend en outre la détermination si un dossier de notes existe déjà pour lesdits paramètres spécifiques affichés (314) et
les paramètres spécifiques affichés (314) sont des valeurs représentant un point de tendance dans les données médicales du patient obtenues par un dispositif de surveillance du patient.

11. Procédé selon la revendication 8, où ledit procédé est prévu pour afficher et annoter des données de paramètres médicales obtenues de plusieurs sources ;
ladite activité d'acquisition acquiert des paramètres médicaux (204) associés à un patient sur une base périodique ;
ladite activité de disposition prioritaire se fait en réponse à une première commande d'utilisateur ;
ladite activité d'identification identifie un ensemble spécifique de paramètres de données dans lesdits paramètres médicaux affichés (212) en réponse à une deuxième commande par l'utilisateur ; et
comprenant en outre l'activité consistant à afficher dans une première fenêtre ledit ensemble spécifique de paramètres de données (410) et dans une seconde fenêtre un champ de texte pour annoter les informations de texte (420) correspondant audit ensemble spécifique de paramètres de données en réponse à une troisième commande par l'utilisateur.

12. Procédé selon la revendication 11, comprenant en outre le stockage dans une mémoire d'un dossier de notes représentant lesdits premier et second affichages de fenêtre (218) et la réalisation d'un indicateur associé auxdits paramètres spécifiques affichés (314) indiquant ledit dossier de notes stocké.

13. Procédé selon la revendication 11, où l'étape d'identification d'un ensemble spécifique de paramètres de données (212) dans lesdits paramètres médicaux affichés comprend l'identification d'au moins un parmi (a) un paramètre de signe vital et (b) une donnée d'estampille temporelle pour l'affichage.

14. Appareil selon la revendication 1, où ladite fenêtre comprend en outre une zone pour rentrer des messages texte définis par l'utilisateur.

15. Système selon la revendication 14, comprenant en outre une icône pouvant être sélectionnée par l'utilisateur pouvant être affichée en réponse à la création par l'utilisateur d'un dossier de notes pour indiquer qu'un dossier de notes a été créé, ledit dossier de notes représentant ledit message texte (218) entré par l'utilisateur et lesdits paramètres spécifiques affichés identifiés (314).
